# EUROPEAN PATENT SPECIFICATION

(11) **EP 1 725 193 B1**
(45) Date of publication and mention of the grant of the patent: **30.09.2009**
(21) Application number: 05708952.6
(22) Date of filing: 05.03.2005
(51) Int. Cl.: A61F 5/00, B29C 45/16

(54) **CLOSURE SYSTEM FOR TUBULAR ORGANS**
VERSCHLUSSSYSTEM FÜR RÖHRENFÖRMIGE ORGANE
SYSTÈME DE FERMETURE POUR ORGANES TUBULAIRES

(30) Priority: 08.03.2004 WO PCT/CH2004/000136
(43) Date of publication of application: 29.11.2006
(73) Proprietor: Allergan Medical S.A., Ecublens (VD) (CH)
(72) Inventor: BACHMANN, Michel, 1126 Vaux-sur-morges (CH); IMBERT,Christian ENDOART S.A., CH-1015 Lausanne (CH); JORDAN, Alain ENDOART S.A., CH-1015 Lausanne (CH)
(74) Representative: Vossius & Partner
(86) International application number: PCT/IB2005/050822
(87) International publication number: WO 2005/087147

(56) References cited:
- WO-A-02/096326

## Description

### Field of the invention

The present invention relates to surgical devices for adjusting the diameter of tubular organs such as the esophagus, the stomach, the colon or the urethra. Such devices may be used as sphincters (e.g. anal or urinary sphincter) or for the control of obesity.

It more precisely relates to surgically implantable adjustable rings for encircling said tubular organs.

### State of the art

Surgical devices for adjusting the diameter of tubular organs are disclosed in patent documents US 5 658 298, US 5 601 604, FR 2 823 663, WO 01185071 and WO 03/059215.

The device disclosed in WO 03/059215 has a ring shape which comprises a first and second end parts and which is designed to be closed around a tubular organ towards its two end parts by a closure system to adjust the diameter of said tubular organ by forming a loop, the first end part forming a sleeve having a first and second open end parts and which is designed to receive the ring second end part, the sleeve main axis being defined along a direction which is substantially perpendicular to the main direction of the ring first end part, the ring second part furthermore comprising a locking protrusion adapted to hold the border of the sleeve second end part and thereby to secure the ring in a closed position.

### Summary of the invention

An object of the present invention is to provide an improved closure system for the previous cited prior art devices.

This and other objects are achieved with the device as defined in claim 1.

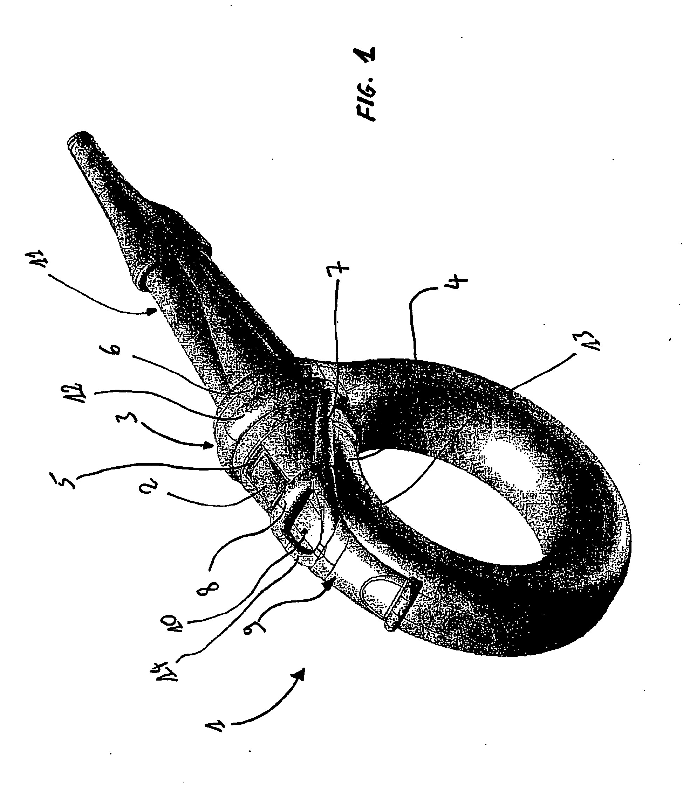

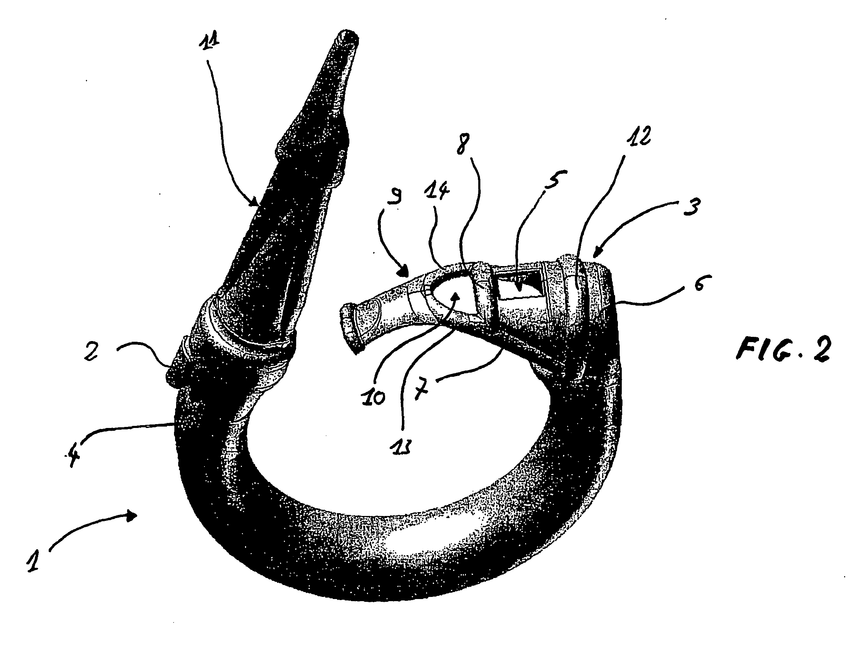

An embodiment of the invention will be discussed in a more detailed way here below together with figures 1 and 2.

The adjustable ring **1** comprises a first **3** and a second **4** end parts.

Any suitable material can be used with the ring **1,** e.g. a biocompatible elastomeric material. The external part of the ring **1** can be more rigid than the internal part, this later one having an internal diameter which can be adjusted. The first end part **3** forms a sleeve which is designed to receive the second end part **4.**

The second end part **4** has an extension **11** which contains adjusting means, for instance a wire which can be pulled or pushed in order to adjust the ring **1** diameter.

The sleeve **3** has a first end part **6** which is reinforced by a flange **12** and a second end part **7** which contains a hole **5** designed to receive and efficiently retain a protrusion **2** which is fixed to the ring second end part 4.

For closing or opening the ring **1** the sleeve second end part 7 is provided with an extension forming a flexible tab **9.**

The tab **9** contains a hole **10** situated close to the sleeve hole **5.** The presence of the hole **10** in the tab **9** provides several advantages, in particular by preventing the accidental opening of the closure system when the tab **9** has to support forces which tend to fold the tab **9** in the direction of the extension **11.** The forces may be due to the movement of the patient or the organs of the patient or to the fluid or bolus passing through the tubular organ. The zone between both holes **5,10** is reinforced by a flange **8.** The other sides of the tab hole **10** are also reinforced by flanges **13,14.**

The protrusion 2 shape is designed to closely match the flange 8 shape.

The invention is of course not limited to the above cited example.

For instance, the hole **10** can be replaced by a portion being more flexible than the remaining part of the tab **9.**

Such a more flexible portion can be obtained by different ways, for example in making the portion thinner than the tab.

The invention can be used for different uses, for instance as a sphincter or as a gastric ring.

## Claims

1. Surgically implantable adjustable ring (1) comprising first (3) and second (4) end parts and which is designed to be closed around a tubular organ towards its two end parts (3,4) by a closure system (2,5) to adjust the diameter of said tubular organ by forming a loop, the first end part (3) forming a sleeve having first (6) and second (7) open end parts and which is designed to receive the ring second end part (4), the sleeve main axis being defined along a direction which is substantially perpendicular to the main direction of the ring first end part (3), the ring second end part (4) furthermore comprising a locking protrusion (2) adapted to hold the sleeve (3) and thereby secure the ring in a closed position, **characterized by** the fact that the sleeve (3) comprises a hole (5) designed to receive said locking protrusion (2).

2. Adjustable ring according to claim 1 wherein the sleeve second end part (7) contains said hole (5) and partially covers the ring second end part (4).

3. Adjustable ring according to claim 2 comprising a reinforcement (8), for instance a flange, situated on at least the hole side which is in close contact with the protrusion (2) when the ring (1) is closed.

4. Adjustable ring according to anyone of the previous claims comprising a tab (9) extending from the sleeve second end part (7).

5. Adjustable ring according to claim 4 wherein the tab (9) comprises a flexible portion, being more flexible than the remaining part of the tab, which is situated close to said sleeve hole (5), in such a way as to prevent an accidental opening of the closure system.

6. Adjustable ring according to claim 5 wherein said flexible portion comprises a hole (10).

## Patentansprüche

1. Chirurgisch implantierbarer regulierbarer Ring (1) mit ersten (3) und zweiten (4) Endabschnitten, der dazu vorgesehen ist, ein röhrenförmiges Organ mit einem Verschlusssystem (2, 5) an seinen beiden Endabschnitte (3, 4) zu umschließen, um den Durchmesser des röhrenförmigen Organs durch Ausbilden einer Schlinge zu regulieren, wobei der erste Endabschnitt (3) eine Hülse mit einem ersten (6) und zweiten (7) offenen Endabschnitt bildet, der dazu vorgesehen ist, den zweiten Endabschnitt (4) des Rings aufzunehmen, die Hauptachse der Hülse entlang einer Richtung definiert ist, die im Wesentlichen senkrecht zur Hauptrichtung des ersten Endabschnitts (3) des Rings ist, der zweite Endabschnitt (4) des Rings ferner einen Verriegelungsvorsprung (2) aufweist, um die Hülse (3) zu halten und **dadurch** den Ring in einer geschlossenen Position zu sichern, **dadurch gekennzeichnet, dass** die Hülse (3) ein Loch (5) zur Aufnahme des Verriegelungsvorsprungs (2) aufweist.

2. Regulierbarer Ring nach Anspruch 1, wobei der zweite Endabschnitt (7) der Hülse das Loch (5) enthält und teilweise den zweiten Endabschnitt (4) des Rings abdeckt.

3. Regulierbarer Ring nach Anspruch 2, mit einer Verstärkung (8), zum Beispiel mit einem Flansch, der sich zumindest auf der Lochseite befindet, die sich in engem Kontakt mit dem Vorsprung (2) befindet, wenn der Ring (1) geschlossen ist.

4. Regulierbarer Ring nach einem der vorhergehenden Ansprüche, mit einer Lasche (9), die sich von dem zweiten Endabschnitt (7) der Hülse erstreckt.

5. Regulierbarer Ring nach Anspruch 4, wobei die Lasche (9) einen flexiblen Abschnitt aufweist, der flexibler als der restliche Abschnitt der Lasche ist, die sich in der Nähe des Lochs (5) der Hülse befindet, um eine versehentliche Öffnung des Verschlusssystems zu verhindern.

6. Regulierbarer Ring nach Anspruch 5, wobei der flexible Abschnitt ein Loch (10) aufweist.

## Revendications

1. Anneau réglable implantable de façon chirurgicale (1) comprenant des première (3) et deuxième (4) parties d'extrémité et qui est conçu pour être fermé autour d'un organe tubulaire vers ses deux parties d'extrémité (3, 4) par un système de fermeture (2, 5) pour régler le diamètre dudit organe tubulaire en formant une boucle, la première partie d'extrémité (3) formant un manchon ayant des première (6) et deuxième (7) parties d'extrémité ouvertes et qui est conçu pour recevoir la deuxième partie d'extrémité (4) de l'anneau, l'axe principal du manchon étant défini le long d'une direction qui est sensiblement perpendiculaire à la direction principale de la première partie d'extrémité (3) de l'anneau, la deuxième partie d'extrémité (4) de l'anneau comprenant en outre une protubérance de verrouillage (2) adaptée pour maintenir le manchon (3) et ainsi fixer l'anneau dans une position fermée, **caractérisé par le fait que** le manchon (3) comprend un trou (5) conçu pour recevoir ladite protubérance de verrouillage (2).

2. Anneau réglable selon la revendication 1, dans lequel la deuxième partie d'extrémité (7) du manchon comporte ledit trou (5) et couvre partiellement la deuxième partie d'extrémité (4) de l'anneau.

3. Anneau réglable selon la revendication 2 comprenant un renforcement (8), par exemple une bride, situé au moins du côté du trou qui est en contact étroit avec la protubérance (2) quand l'anneau (1) est fermé.

4. Anneau réglable selon l'une quelconque des revendications précédentes, comprenant une patte (9) s'étendant depuis la deuxième partie d'extrémité (7) du manchon.

5. Anneau réglable selon la revendication 4, dans lequel la patte (9) comprend une partie flexible, plus souple que la partie restante de la patte, qui est située près dudit trou de manchon (5), de façon à prévenir l'ouverture accidentelle du système de fermeture.

6. Anneau réglable selon la revendication 5, dans lequel ladite partie flexible comprend un trou (10).
